# EUROPEAN PATENT SPECIFICATION

(11) **EP 2 185 101 B1**
(45) Date of publication and mention of the grant of the patent: **24.07.2013**
(21) Application number: 08776068.2
(22) Date of filing: 28.07.2008
(51) Int. Cl.: A61P 15/08, A61K 38/46, C12N 15/873

(54) **FERTILISATION PROTEIN**
FERTILISATIONSPROTEIN
PROTÉINE DE FERTILISATION

(30) Priority: 08.08.2007 GB 0715325
(43) Date of publication of application: 19.05.2010
(73) Proprietor: University College Cardiff Consultants Limited, Cardiff CF24 0DE (GB)
(72) Inventor: LAI, Francis, Anthony, Cardiff CF14 4XN (GB)
(74) Representative: Fyles, Julie Marie
(86) International application number: PCT/GB2008/002561
(87) International publication number: WO 2009/019433

(56) References cited:
- WO-A-03/035678
- WO-A-2008/063577
- FUJIMOTO S ET AL: "Mammalian phospholipase Czeta induces oocyte activation from the sperm perinuclear matrix" DEVELOPMENTAL BIOLOGY, ACADEMIC PRESS, NEW YORK, NY, US, vol. 274, no. 2, 15 October 2004 (2004-10-15), pages 370-383, XP004571330 ISSN: 0012-1606
- KOUCHI ZEN ET AL: "Recombinant phospholipase Czeta has high Ca2+ sensitivity and induces Ca2+ oscillations in mouse eggs." JOURNAL OF BIOLOGICAL CHEMISTRY, vol. 279, no. 11, 12 March 2004 (2004-03-12), pages 10408-10412, XP002509673 ISSN: 0021-9258 cited in the application
- SAUNDERS C M ET AL: "PLC zeta: a sperm specific trigger of Ca2+ oscillation in eggs and embryo development" DEVELOPMENT, COMPANY OF BIOLOGISTS, CAMBRIDGE, GB, vol. 129, 1 January 2002 (2002-01-01), pages 3533-3544, XP002238166 ISSN: 0950-1991 cited in the application
- SWANN ET AL: "PLCzeta(zeta): A sperm protein that triggers Ca<2+> oscillations and egg activation in mammals" SEMINARS IN CELL AND DEVELOPMENTAL BIOLOGY, ACADEMIC PRESS, GB, vol. 17, no. 2, 1 April 2006 (2006-04-01), pages 264-273, XP005560846 ISSN: 1084-9521
- NAZ R K ET AL: "ENHANCEMENT OF SPERM FUNCTION FOR TREATMENT OF MALE INFERTILITY" JOURNAL OF ANDROLOGY, J.B. LIPPINCOTT CO., PHILADELPHIA, US, vol. 16, no. 5, 1 September 1995 (1995-09-01), pages 384-388, XP001061887 ISSN: 0196-3635

## Description

During fertilization of human oocytes, the sperm induces a long-lasting series of repetitive intracellular Ca²⁺ transients, (Ca²⁺ oscillations). Such Ca²⁺ oscillations are also observed in human or mouse oocytes injected with human sperm during intracytoplasmic sperm injection (ICSI). These Ca²⁺ oscillations are a conserved feature of oocyte activation in all mammals and have been extensively studied in mouse and hamster oocytes. Oscillations in Ca²⁺ are responsible for causing the major events of oocyte activation such as cortical granules exocytosis, zona hardening and polyspermy block, resumption of second meiotic division, and initiation of the first mitotic cell cycle. Furthermore, in mouse and rabbit oocytes the number, frequency, as well as the amplitude and duration of Ca²⁺ pulses, influence the efficiency of activation and may regulate later developmental events, such as the ratio of cell types in the blastocyst, or the embryo size and morphology after implantation.

Artificial, or parthenogenetic, activation of mammalian oocytes can be achieved by a range of stimuli that cause a rise in intracellular Ca²⁺ levels, such as ethanol, Ca²⁺ ionophores, electroporation in the presence of Ca²⁺, or incubation in Sr²⁺ media. Ca²⁺ elevating agents, in combination with drugs such as cycloheximide or 6-DMAP, are also used to trigger oocyte activation after nuclear transfer procedures designed to generate cloned embryos. Such parthenogenetic activating agents have been used to stimulate development after failed fertilization in some cases of human ICSI. It has been suggested that this approach could be employed to overcome some cases of failed fertilization due to male factors. There is at least one report of a pregnancy and one of a live birth after chemical activation of oocytes that previously had failed to fertilize during ICSI. However, the success rate of such procedures is unclear. In addition, the stimulation by Ca²⁺ ionophore does not mimic the type of stimulation that occurs during normal fertilization since the characteristic sperm-induced Ca²⁺ oscillations are not observed with artificial activation protocols. The application of ionophores, ethanol or single electric pulses all generate a single large Ca²⁺ increase in the mammalian oocyte. Incubation in media containing Sr²⁺ ions does cause repetitive Ca²⁺ oscillation in rodent oocytes, and is the parthenogenetic agent of choice for nuclear transfer work. However, the pattern of Sr²⁺-induced Ca²⁺ oscillations is different from that at fertilization. Also, for reasons that are not clear, Sr²⁺ is not generally effective in activating oocytes from other non-rodent species, and it has not been shown to cause Ca²⁺ oscillations in human oocytes. Hence, it would be useful to apply a stimulus that mimics the endogenous series of Ca²⁺ oscillations observed at fertilization, since preliminary experimental studies have found that imposing at least three, as opposed to one, transient Ca²⁺ increase is a more effective way of inducing development after failed ICSI in human oocytes.

Substantial evidence now suggests that during normal fertilization, and after ICSI, the sperm causes Ca²⁺ oscillations by the introduction of a soluble protein factor into the ooplasm. We recently demonstrated that the soluble protein factor in sperm extracts is a novel type of phospholipase C called PLCζ(zeta) (Saunders *et al.,* 2002). PLCζ is sperm-specific and injecting this protein, or the cognate cRNA, can produce Ca²⁺ oscillations similar to those generated by the sperm at fertilization in the mouse (Saunders *et al.,* 2002; Kouchi *et al.,* 2004). PLCζ has also been shown to be the protein responsible for oocyte activation during ICSI in the mouse (Fujimoto *et al.,* 2004), and that a reduction in the level of PLCζ protein in sperm via RNA interference can lead to a decrease in the number of Ca²⁺ oscillations and to a reduction in fertility (Knott *et al.,* 2005). These data all suggest that PLCζ is the sperm protein that triggers mammalian development.

PLCζ is found in a wide range of mammalian species and has been demonstrated to be present in human sperm. Injection of cRNA encoding human PLCζ (hPLCζ) into mouse or human oocytes that had failed to fertilize, triggers a long lasting series of Ca²⁺ oscillations, and can lead to oocyte activation and embryo development to the blastocyst stage. The successful application of this novel, sperm-specific hPLCζ therefore represents an alternative parthenogenetic activation protocol. However, although previous studies have shown an effective way of introducing hPLCζ into oocytes, we have surprisingly found there is a precise range of concentrations in which PLCζ can effectively trigger both egg activation and, importantly, embryo development.

### Statements of Invention

Accordingly there is therefore disclosed an effective amount of human PLCζ to enable development after fertilisation of a human oocyte characterised in that said effective amount of human PLCζ is between 170 and 410 femtograms per human oocyte.

Human PLCζ is described in a number of publications, including our earlier application, WO 03/035678, which gives sequences for the polypeptide and the DNA encoding it and describes the activity of the polypeptide in triggering egg activation. However, that document does not contain any teaching relating to the concentration range at which human PLCζ is effective.

In the present application, the term "human PLCζ" refers to one of the following: a polypeptide of SEQ ID NO: 2
a derivative of the polypeptide of SEQ ID NO: 2;
a polypeptide which is substantially homologous to the sequence of SEQ ID NO: 2;
a polypeptide which hybridises to SEQ ID NO: 2 under stringent conditions;
a polypeptide encoded by the nucleic acid sequence of SEQ ID NO: 1; or a mixture of any of the above.

The skilled person will appreciate that homologues or derivatives of the proteins or polypeptides of the invention will also find use in the context of the present invention. Thus, for instance proteins or polypeptides which include one or more additions, deletions, substitutions or the like are encompassed by the present invention. In addition, it may be possible to replace one amino acid with another of similar "type". For instance, replacing one hydrophobic amino acid with another. One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of analysis are contemplated in the present invention.

By "substantially homologous" herein is meant that the nucleic acid sequence has at least 70% identity of its nucleotide bases with those of sequence (a), in matching positions in the sequence. A further 10% of its nucleotide bases may comprise conservative substitutions (with similar bases), and therefore the sequence has at least 80% overall homology. More preferred are sequences having at least 80% identity with the sequence (a) and about 90% overall homology. Such homologous sequences encode a protein having substantially the same biological activity as the proteins of the invention.

Reference herein to stringent conditions refers to a temperature of incubation of at least 45°C but more suitably to 65°C and/or washing the annealed molecules using a salt solution having an ionic strength of from 1.0M sodium chloride to 0.02M sodium chloride.

References to a protein enabling "development" of human oocytes after fertilisation refer to the ability of the protein to induce a long-lasting series of Ca²⁺ oscillations, which in turn lead to oocyte activation and embryo development.

According to a first aspect of the invention there is provided a composition comprising human PLCζ and a pharmaceutically acceptable excipient or carrier, wherein the composition is a fertilisation fluid for injecting into a human oocyte and said fluid is characterised in that the injected volume contains between 170 and 410 femtograms of human PLCζ.

The term "pharmaceutically acceptable excipient or carrier" as used herein should be taken to mean any inert, non-toxic, solid or liquid filler, diluent or encapsulating material, or other excipient, which does not react adversely with the active ingredient(s) or with a patient. Such formulations and carriers are well known in the art.

There is further disclosed human PLCζ for use in medicine, particularly in the treatment of infertility, characterised in that the human PLCζ is provided in an amount of between 170 and 410 femtograms of human PLCζ per human oocyte.

Accordingly there is disclosed the use of human PLCζ in the manufacture of a medicament to treat infertility wherein the medicament is formulated so that the amount of human PLCζ, when transferred to an oocyte, is between 170 and 410 femtograms.

The medicament or composition, for example the fertilisation fluid, may be used to treat a sperm sample or an oocyte for an *in vitro* fertilisation method, for example in combination with ICSI.

The elucidation of an effective concentration range of human PLCζ leads to the possibility of effective treatments for male infertility and therefore the disclosure also teaches a method for treating male infertility comprising:
a) using a sample of sperm obtained from a human male wherein the concentration of human PLCζ per sperm cell has been determined to be less than 170 femtograms;
b) supplementing the amount of human PLCζ so that it falls within a range of between 170 and 410 femtograms;
c) using the supplemented sperm cell to activate or fertilise a selected oocyte.

Reference herein to the term activate includes reference to the enablement of development following fertilisation.

In a preferred method of the disclosure supplementing the sperm cell to increase the amount of human PLCζ may be undertaken by directly injecting or inserting a suitable amount of PLCζ protein therein or, alternatively, genetic manipulation may be performed such that the sperm cell expresses an increased amount of human PLCζ (over the above that it would normally express without genetic manipulation), so that the total amount of human PLCζ expressed by the sperm cell is between 170 and 410 femtograms.

The invention further provides an *in vitro* fertilisation method comprising fertilising a human oocyte with sperm obtained from a human male wherein the concentration of human PLCζ per sperm cell has been determined to be less than 170 femtograms; and injecting the oocyte with human PLCζ such that the amount of human human PLCζ in the oocyte after injection is between 170 and 410 femtograms.

In this aspect of the invention, the oocyte may be injected with human PLCζ before, after or simultaneously with the sperm.

It is often preferable to carry out the fertilisation step using a method such as ICSI.

Typically, the PLCζ used in the method is in a formulation such as the fertilisation fluid discussed above.

The elucidation of an effective concentration range of human PLCζ also enables diagnostic methods to be developed. Therefore, according to a further aspect of the invention there is provided a diagnostic or screening method for determining the fertility status of a human male comprising:
a) using a sample of sperm obtained from said human male to determine the concentration of human PLCζ contained therein by:
b) measuring the amount of human PLCζ in each sperm cell and where the concentration is less than 170 femtograms;
c) concluding that the male is likely to be infertile.

As set out above, males in whom the concentration of human PLCζ per sperm cell is above 410 femtograms may also suffer from infertility and therefore the disclsoure also teaches a method for treating male infertility comprising:
a) using a sample of sperm obtained from a human male wherein the concentration of human PLCζ per sperm cell has been determined to be more than 410 femtograms;
b) blocking the activity of a fraction of said PLCζ so that the active amount of PLCζ able to promote development of a female oocyte to the blastocyst stage and beyond is between 170 and 410 femtograms;
c) using the blocked sperm cell to activate or fertilise a selected oocyte.

In a preferred embodiment of the disclsoure supplementing the sperm cell to decrease the amount of human PLCζ is undertaken by genetic manipulation such that the sperm cell expresses a decreased amount of human PLCζ (below that it would normally express without genetic manipulation), so that the total amount of human PLCζ expressed by the sperm cell is less than 410 femtograms.

According to a further aspect of the invention there is provided a diagnostic or screening method for determining the fertility status of a human male comprising:
a) using a sample of sperm obtained from said human male to determine the concentration of human PLCζ contained therein by:
b) measuring the amount of human PLCζ in each sperm cell and where the concentration is more than 410 femtograms;
c) concluding that the male is likely to be infertile.

In all the above aspects of the invention the range of PLCζ is expressed as 170-410 femtograms.

The invention will now be described by way of example only with reference to the following Figures wherein:

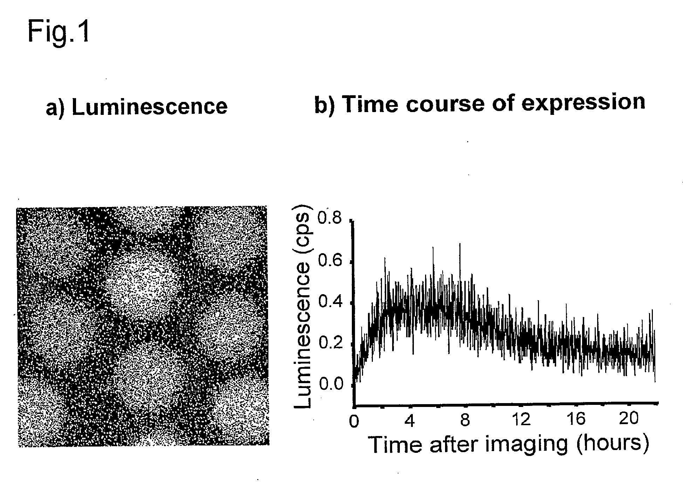

**Figure 1**. In a) is shown a luminescence image of typical group of mouse oocytes that had been injected with hPLCζ -luc cRNA and placed in luciferin containing media. The image consists of the integrated photon counts accumulated over a period of 20 hours. In b) the trace illustrates an example of the luminescence (in photon counts per second) from one of mouse oocytes in part (a);

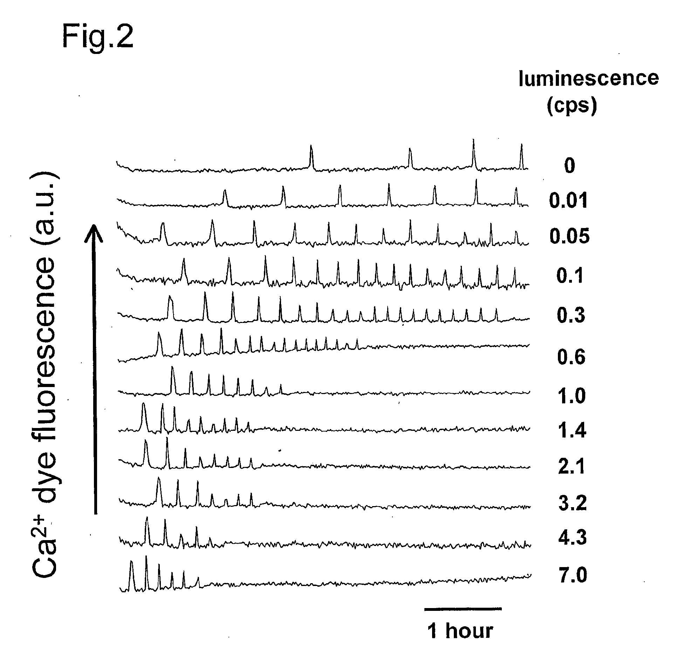

**Figure 2**. The pattern of Ca²⁺ oscillations caused by different amounts of hPLCζ -luc expression in individual mouse oocytes. The fluorescence of the Ca²⁺ sensitive dye Oregan Green BAPTA is shown on the y-axis in arbitrary units (a.u.). The Ca²⁺ oscillation pattern is then shown with 12 traces for 12 different oocytes, each with a different level of hPLCζ -luc expression. The luminescence expression levels in counts per second (cps) are indicated for each associated fluorescence trace that indicates the Ca²⁺ levels;

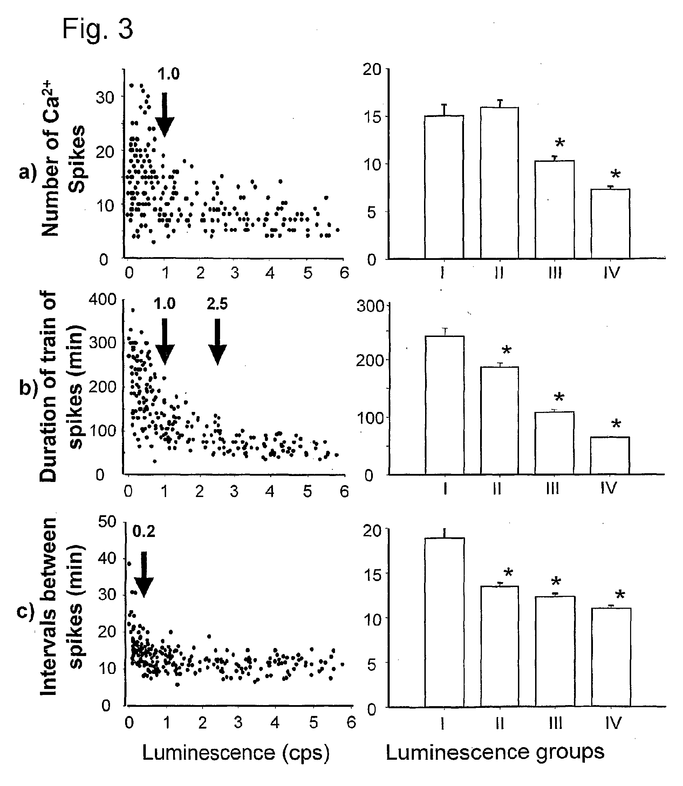

**Figure 3**. The relationship between Ca²⁺ oscillations and hPLCζ-luc expression. A total of 233 oocytes were imaged, analyzed and plotted as a scatter of dots which show that there were distinct trends in the number, duration and intervals of Ca²⁺ increases that correlated with the hPLCζ-luc expression levels in individual oocytes. According to the changes of calcium oscillation pattern (left), cps 0.2, 1.0 and 2.5 (arrows) were thought to be the transition points and then the levels of hPLCζ-luc expression were classified into four different ranges; I (0-0.2cps), II (0.2-1.0cps), III (1.0-2.5cps) and IV (2.5-6cps). (Range I, II, III, IV. right). The bar charts on the right hand side show that there are statistically significant differences pattern of Ca²⁺ oscillations (number of spikes, duration of train, or the intervals between spikes) as the expression levels of hPLCζ-luc increases (the * indicates a significant difference with P<0.05.);

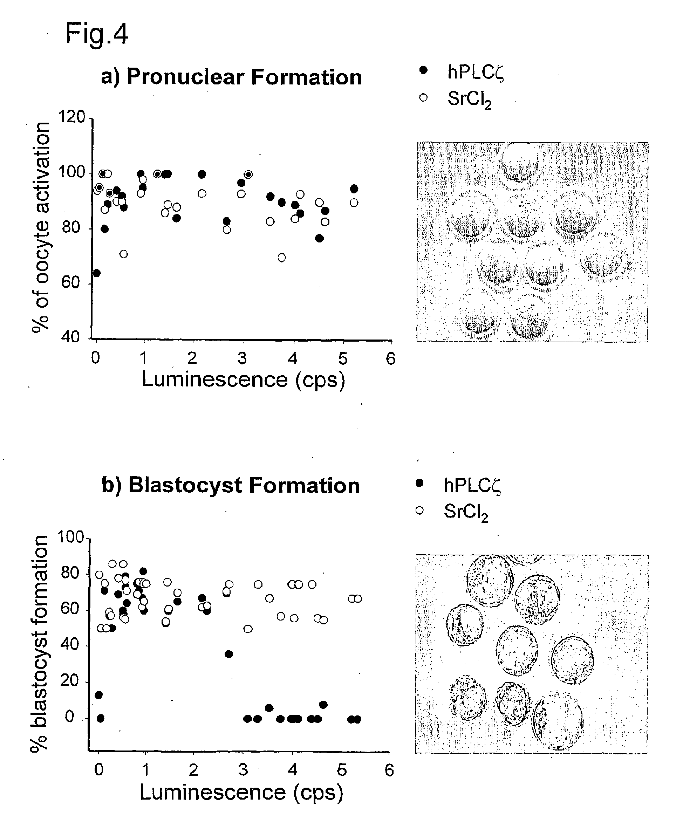

**Figure 4**. The effects of hPLCζ-luc cRNA injection on oocyte activation and development. In a) the rate of oocyte activation is scored as pronuclear formation rate divided by the number of injected oocytes. The luminescence level is plotted for each group of oocytes injected with hPLCζ-luc (closed circles). The open circles represent the activation rates for groups of Sr²⁺ treated oocytes that were not luminescent but that were treated in parallel on the same day. In b) the development rate is plotted as the number of blastocysts formed divided by the number of activated oocytes. As with a) this is done for hPLCζ-luc activated embryos at the range of luminescence values plotted on the x-axis (closed circles), and for a parallel group of (non-luminescent) Sr²⁺ activated embryos (open circles). The right hand panels for a) and b) show examples of hPLCζ-luc activated oocytes and blastocysts respectively;

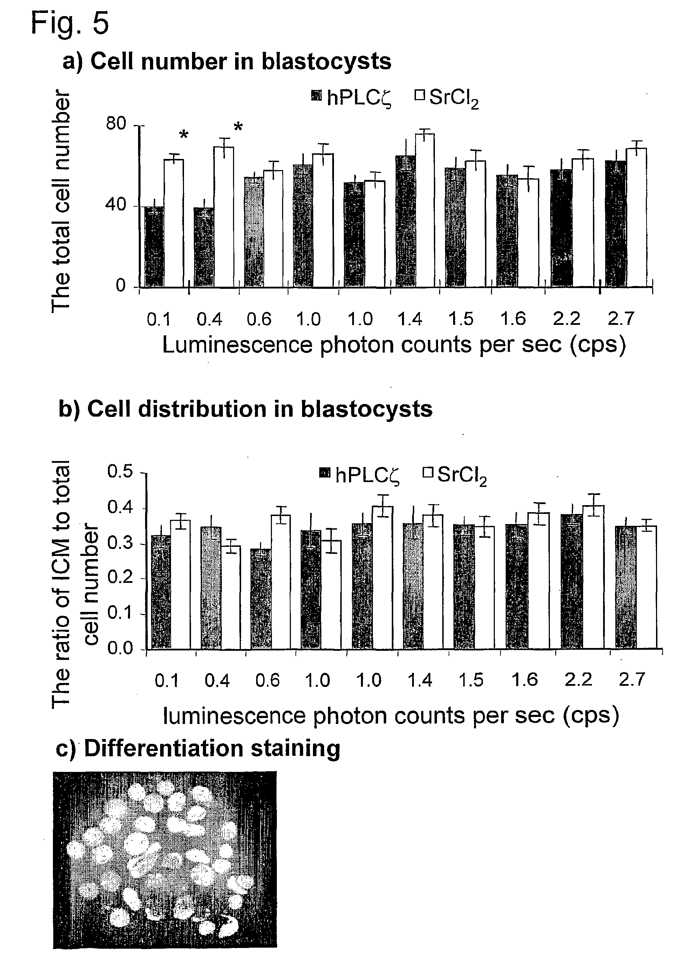

**Figure 5****.** The distribution of cells in blastocysts analyzed by differentiation staining. The plots in a) show the total cell number and in b) the ratio of ICM cells to trophoblastic cells in blastocyst activated by hPLCζ-luc. Each bar represents a group of embryos that had been activated by a different expression level of hPLCζ-luc as plotted on the x-axis. The number / distribution of cells for each group of Sr²⁺- activated embryos is shown alongside the parallel PLCζ group. In each experiment, at least 10 blastocysts were analyzed, and the * indicates a significant difference with P<0.05. In c) a typical image is shown for an hPLCζ-luc activated blastocyst with the ICM cells and trophoblastic cells stained in blue and pink respectively;

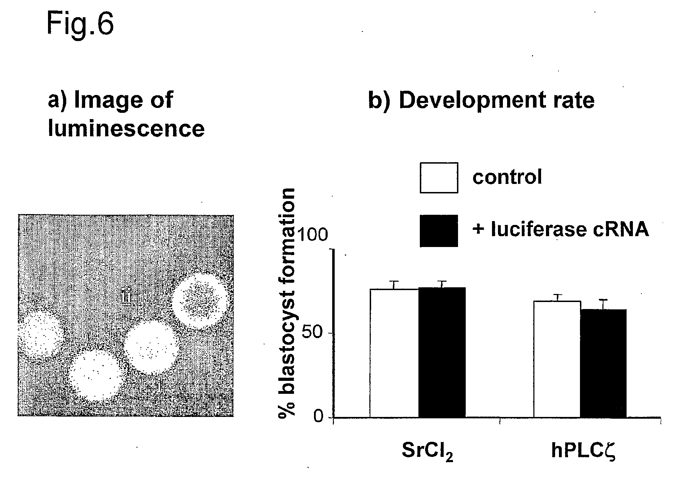

**Figure 6****.** The development of embryos injected with control luciferase cRNA. The panel a) shows an image of some oocytes injected with luciferase RNA (i) that has 23 cps, and with a typical amount of hPLCζ-luc cRNA (ii) which showed 0.83cps. The image is from integrated counts over 20 minutes. In b) are shown the development rates of embryos activated by either Sr²⁺ or by hPLCζ-luc cRNA (open boxes). In each case development was compared with embryos that had also been injected with excess of luciferase cRNA (filled boxes);

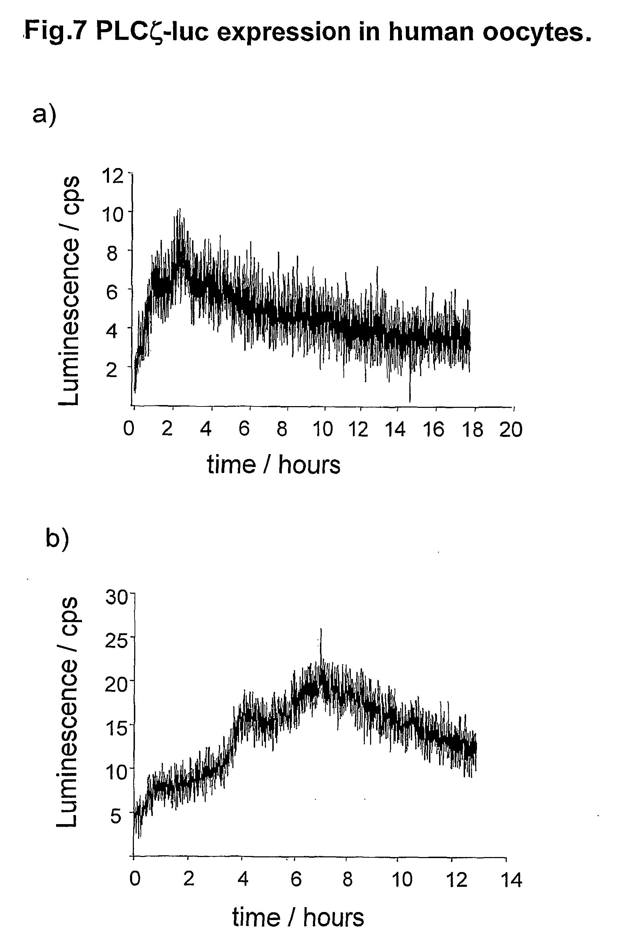

**Figure 7****.** The Figure shows luminescence (in photon counts per second) following the injection of human PLCζ, tagged with luciferase, in human ooyctes. The difference traces in Figures 7A and 7B illustrate that the pattern of expression of human PLCζ was somewhat variable;

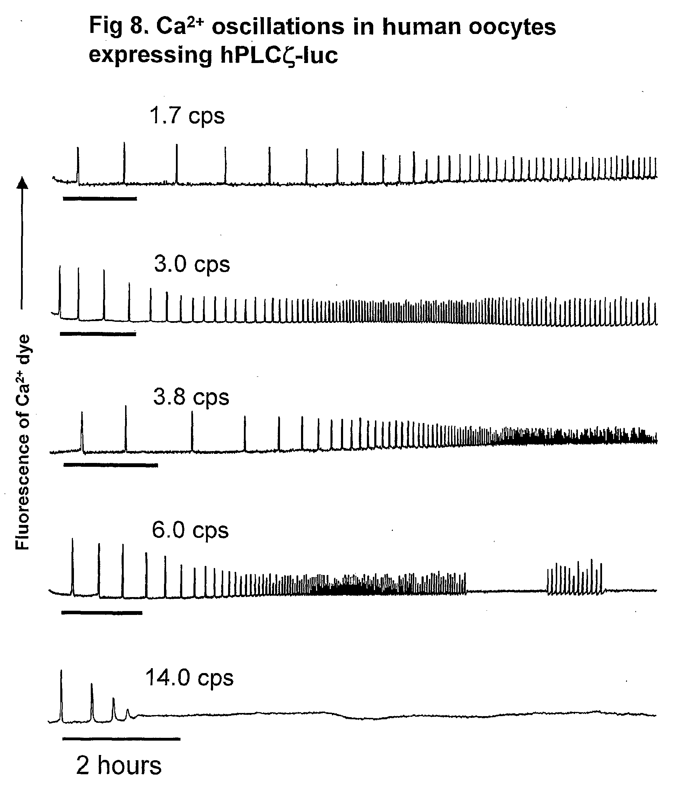

**Figure 8****.** This shows the pattern of calcium oscillations caused by different amounts of hPLCζ-luc expression in individual human oocytes. The fluorescence of the calcium sensitive dye is shown on the Y-axis. The calcium oscillation pattern is shown in 5 traces for 5 difference human oocytes displaying different levels of hPLCζ-luc expression; and

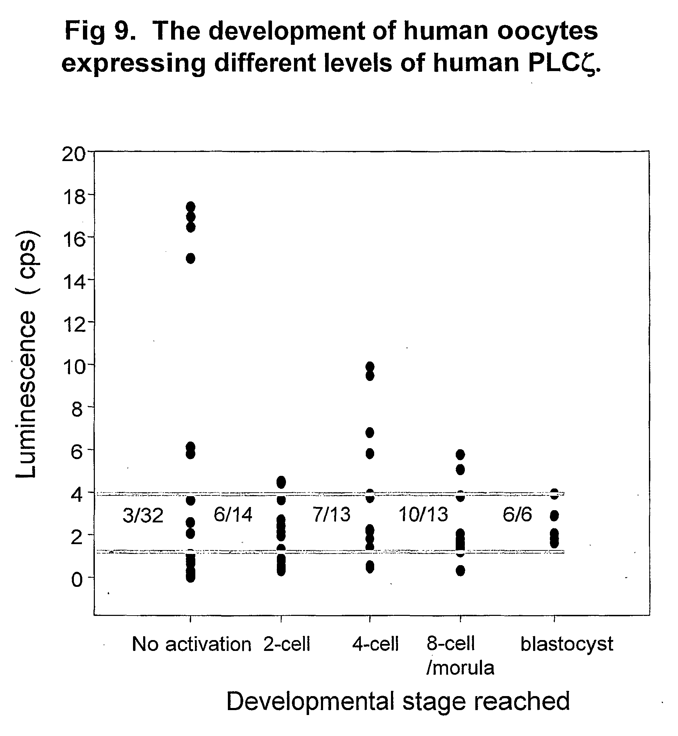

**Figure 9****.** This shows the development of human eggs expressing different levels of human PLCζ. The development stages are recorded along the X-axis for a range of human eggs each expressing a different level of human PLCζ. In a range of expression level of human PLCζ of 1.2-4 cps the cleavage, 4 cell, 8 cell and blastocyst rate is 29/32, 23/32, 16/32 and 6/29.

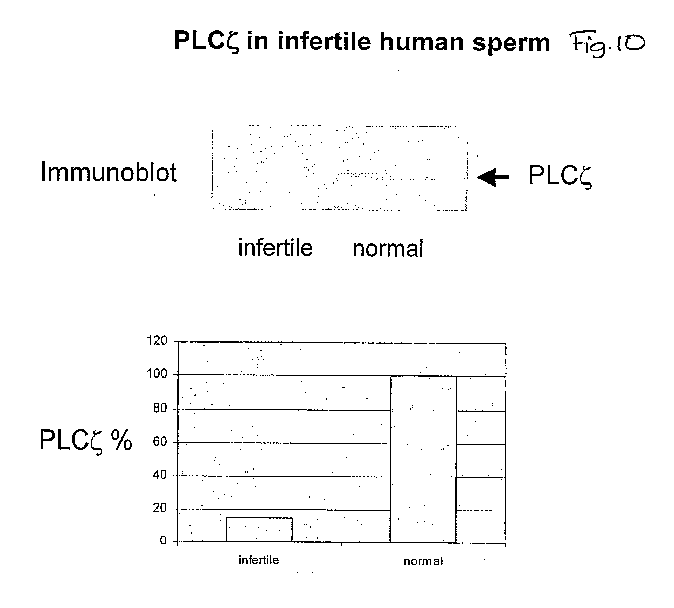

**Figure 10** Upper panel: Immunoblot showing the ∼70kD protein band corresponding to the mobility of human PLC-zeta, indicated by an arrow. Lower panel: Densitometric analysis of the immunoreactive band indicating the relative content of PLC-zeta in infertile human sperm (15%) relative to normal human sperm (100%).

### Example 1

### Materials and Methods

### Handling and microinjection of oocytes.

MF1 female mice about 4-6 weeks were superovulated as described previously (Saunders *et al.,* 2002; Larman *et al.,* 2004). Oocytes were released from the oviduct ampulae into M2 medium (Sigma-Aldrich, Poole, Dorset, UK) with a needle 13-15 hr post-hCG followed by removal of the cumulus mass by 0.3mg/ml hyaluronidase. Complementary RNA encoding the 608-residue human PLC, tagged via the C-terminus with firefly luciferase (hPLCζ-luc), was prepared as described previously (Nomikos et. al. 2005). Microinjection procedures were carried out as previously described (Saunders *et al.,* 2002). Oocytes were injected to 3-5% egg volume with a solution containing different concentrations of hPLCζ-luc cRNA (0.05-0.5 µg/µl) and 1 mM Oregon Green BAPTA dextran (Invitrogen Ltd, Paisley, UK). In the control experiment, 0.5 µg/µl luciferase RNA was injected into oocytes before they were activated by SrCl₂ or hPLCζ-luc cRNA injection.

### Measurement of intracellular Ca²⁺ and luciferase expression

Some of the injected oocytes were placed in a chamber with M2 medium (Sigma Aldrich, Poole, Dorset, UK) containing 1 mM luciferin, on the temperature-controlled stage of an inverted microscope. Ca²⁺ oscillations were monitored by measuring the fluorescence of Oregon Green BAPTA dextran and luciferase expression was monitored by the luminescence. These measurements were both carried out on the same sets of oocytes using a Zeiss Axiovert S100 microscope with light from the stage directed towards a cooled intensified CCD camera (ICCD) with a bialkali-type photocathode-based intensifier cooled to 10°C. The microscope and ICCD camera were placed inside a custom-made dark box. This photon counting camera, dark box, and associated software was supplied by Photek Ltd (St Leonards on Sea, East Sussex, UK). In most experiments, the fluorescence was recorded first by exposing oocytes to excitation light (450-490nm) and reducing the sensitivity of the ICCD camera to 10%, and then the luminescence was recorded by removing the excitation light and switching the ICCD camera to maximum sensitivity. The luminescence values in experiments represent the absolute number of measured photon counts per second, whereas the intensity of fluorescence is displayed in arbitrary units of intensity. The levels of luciferase protein corresponding to a level of luminescence was estimated by injecting oocytes with known amounts of recombinant luciferase protein (Sigma Aldrich, Poole, Dorset, UK) and then measuring the luminescence of these oocytes under the same conditions as those injected with hPLCζ-luc cRNA.

### Culture and analysis of embryos.

On each experimental day, some of the hPLCζ-luc-injected oocytes were imaged and some from the same batch were put into KSOM media (Summers *et al*., 2000), containing 5 µg/ml cytochalasin B for 6 hr. A separate batch of oocytes that were not injected with hPLCζ-luc were activated by 10 mM SrCl₂ in Ca²⁺-free KSOM medium for 4 hr. After the pronuclei formation was checked, both types of activated oocytes were cultured in KSOM medium at 37°C in a 5% CO₂ incubator for 96 hr. All the resulting blastocysts were incubated in 0.5% pronase to remove the zona pellucida. After washing in M2 medium, blastocysts were incubated in 10% rabbit anti-mouse whole serum for 30 min, washed again with M2 medium, and then incubated in M2 medium containing 20% guinea pig complement, 30 µg/ml propidium iodide and 10 µg/ml Hoechst 33342 for 15 min. The embryos were rinsed quickly and mounted in glycerol onto a glass slide. The data were expressed as means ± SE. To evaluate the statistical significance of differences between groups, we applied the Student's *t*-test to test for mean comparisons. A *P* level of ≤0.05 was considered statistically significant. All chemicals not otherwise specified were obtained from Sigma-Aldrich (Poole, Dorset, UK).

### Microinjection of human oocytes

Human oocytes were obtained from the local IVF clinic. The oocytes were of two types. So called 'fresh' human oocytes were collected from women undergoing follicle reduction that is sometimes necessary as a result of an over-response to hormonal stimulation for interuterine insemination. So called 'aged' human oocytes were oocytes that had failed to show any signs of fertilization after normal IVF procedures and those collected from the IVF clinic >16 hours after initial insemination. Full consent was obtained from the patients for the use of oocytes. Our research project has been approved by the local research ethics committees and by the Human Fertilization and Embryology Authority (research licence number R0161). Oocytes were microinjected with hPLCζ-luc RNA in the same way as described for mouse eggs (Yu et al. 2007). The luminescence from all injected oocytes was measured for 30 mins at 15-18 hours post-injection, by incubating eggs in M2 media containing 1 mM luciferin and placing them on the stage of a Nikon TE2000 microscope equipped with a photon counting camera (Photek ICCD). The amount of luciferase expressed was calibrated by injection of known amounts of recombinant luciferase protein (Sigma-Aldrich) into mouse eggs. After hPLCζ-luc injection of the human oocytes, they were placed in drops of Sidney IVF cleavage medium for 3 days followed by transfer to Sidney IVF blastocyst medium (COOK), both in a 6% CO₂ incubator. Their development was observed intermittently over 5 days. Activation was defined as the appearance of pronuclei. In some cases, oocytes were also injected with the Ca²⁺-sensitive dye, Oregon Green BAPTA dextran, and then the fluorescence (reporting changes in Ca²⁺) and the luminescence was monitored for 15-18 hours by switching between fluorescence and luminescence imaging modes (Campbell and Swann, 2006).

### Results

### Expression of hPLCζ-luciferase and cleneration of Ca²⁺ oscillations in mouse oocytes.

When mouse oocytes were microinjected with hPLCζ-luc cRNA, the luminescence, level (an indicator of luciferase protein concentration) as measured in photon counts per second, started to increase within the first hour and detection of luminescence continued for over a 20 hr period (Fig.1). Fig.1 b shows that the luminescence level gradually accumulated until ∼3 hours after cRNA injection when a plateau level of about 0.4 cps was achieved for about 6 hours, after which there was a gradual decline over ∼3 hr to a luminescence level of ∼0.1 cps. Since Ca²⁺ signaling and oocyte activation generally occurs within 6 hours of injection, in subsequent experiments we measured Ca²⁺ changes in the first 6 hour period post-injection followed by determination of luminescence from the same oocytes for 30 mins in the presence of luciferin to obtain the level of PLCζ-luc protein.

The effects of different amounts of PLCζ-luc protein on activation are shown in Figure 2. Various pipette concentrations of 0.05-0.5 µg/µl of hPLCζ-luc cRNA were injected into groups of mouse oocytes. A total of 233 oocytes were microinjected with cRNA and subsequently monitored for Ca²⁺ oscillations and luciferase expression. Fig.2 shows some typical examples of the distinct patterns of Ca²⁺ oscillations occurring in oocytes due to different levels of hPLCζ-luc expression. Increasing expression levels from 0.01 to 0.3 cps resulted in an enhanced frequency of spikes that was maintained for over 5 hr. However, surprisingly higher expression levels of >0.3 cps caused a cessation of the spikes after 1-3 hr, preceded by a gradual decrease in spike amplitude. The scatter plots in Figure 3 (left column) illustrate the relationship between the pattern of Ca²⁺ oscillations, as indicated by the number, duration and interval of responses, and the level of luciferase expression. Fig.3 also presents a histogram analysis (right column) where patterns of oscillations (number, duration and interval) are grouped according to the increasing level (I-IV) of luciferase expression. Fig.3a identifies expression levels I and II as optimal for spike number, whereas the higher levels III and IV cause a reduction. The duration of the train of the spikes also decreases with increasing expression levels, with maximal duration occurring with I (Fig.3b). From Fig.3c it can be seen that interspike interval also is reduced with increasing hPLCζ-luc expression, and hence there is an increase in the frequency of Ca²⁺ oscillations. Since the duration of the whole train of Ca²⁺ oscillations decreases as the frequency increases there is only a small increase in the number of Ca²⁺ spikes in oocytes with high hPLCζ-luc concentrations. Moreover, with high concentrations of hPLCζ-luc, it is evident that the cessation of Ca²⁺ oscillations at high expression levels (e.g. after 1-2 hr in the 1.0 to 7.0 cps traces in Fig.2) occurs well before the peak of protein expression has occurred (after 3-4 hr in Fig. 1 b). These data support previous studies suggesting that the pattern of Ca²⁺ oscillations is affected by the amount of PLCζ cRNA injected into each oocyte (Saunders *et al.,* 2002; Cox *et al.,* 2002), but it is now evident that the variation in the pattern of Ca²⁺ oscillations is seen over a relatively small range of PLCζ protein concentrations.

We estimated the protein expression level in these hPLCζ-luc-injected oocytes by comparing the amount of light emitted from embryos on the imaging system with that from oocytes injected with known amounts of luciferase protein. We found that 1 cps of luminescence corresponded to about 250fg of luciferase protein. Since Ca²⁺ oscillations were triggered with expression levels as low as 0.01 cps, we can estimate that as little as 2.5fg of hPLCζ-luc protein at 6 hours is associated with Ca²⁺ release. However, since we also know that Ca²⁺ oscillations in these cases started about 1-2 hours after injection (Fig.2) we can estimate that levels of around 1fg of hPLCζ-luc are sufficient to trigger Ca²⁺ release in mouse oocytes.

### Developmental potential of embryos activated by different levels of hPLCζ-luc.

The imaging experiments described above were carried out on a small group of oocytes taken from a larger cohort. For the remaining oocytes we assessed their developmental potential by placing them in KSOM media with cytochalasin B for 6 hours, followed by sustained culture in normal KSOM media. To evaluate the potential of hPLCζ-luc to activate mouse eggs and trigger subsequent development we monitored both pronuclei and blastocyst formation at 6 hours and 96 hours after injection, respectively. We also compared the results from hPLCζ-luc-injected oocytes to the developmental potential of a group of Sr²⁺-activated oocytes that had been collected from the same set of mice on the same day.

Figure 4a shows that at a very low level of PLCζ-luc expression (0.05cps) mouse oocytes can be effectively activated to form pronuclei. Furthermore, over a large range of expression levels (0.05-6cps) nearly all oocytes formed pronuclei at 6 hr after hPLCζ-luc injection (95.9%). This is comparable to the pronuclei formation efficiency observed for SrCl₂ activation (85.7±2.4%), and is also consistent with previous observations showing mouse oocyte activation upon microinjection of various cRNA concentrations of the untagged hPLCζ (Cox *et al.,* 2002). However, we found that the further development of hPLCζ-luc-injected oocytes beyond pronuclei formation at 6 hr was markedly dependent upon the precise level of PLCζ-luc expression. Figure 4b shows the rate of development to the blastocyst stage for PLCζ-luc-injected oocytes was >50% (i.e. similar to Sr²⁺-activated oocytes) only when the level of hPLCζ-luc expression was between narrowly defined limits of 0.12 to 2.7 cps. Most notably, when the expression of hPLCζ-luc was greater than 2.7 cps, the embryos all failed to develop into blastocysts. Most of these high hPLCζ-luc expression embryos (>2.7 cps) arrested at the 2-cell stage with only 38% reaching the 4-cell stage. These data show that there is a wide range of concentrations where hPLCζ-luc can fully activate oocytes (i.e. induce pronuclei formation), but that ability to activate in itself does not guarantee that embryos will develop into blastocysts. These observations suggest that only a specific, narrow window of hPLCζ levels is consistent with successful pre-implantation development.

### Assessments of blastocysts obtained after hPLCζ-luc injection.

We evaluated the blastocyst embryos obtained from hPLCζ-luc injection by analysis of the total cell number, and the cell number ratio between ICM cells and trophoblast cells within the blastocysts. This was done by differential staining (Fig.5), which has been widely used to evaluate the quality of blastocysts (Van Soom *et al.,* 2001). We examined blastocysts derived from ten separate experiments in which the expression levels of hPLCζ-luc ranged between 0.1 and 2.7 cps. The data shown in Fig. 5 demonstrates that all blastocysts obtained after PLCζ-luc injection have a similar ratio of cells allocated between the ICM and the trophectoderm, and this ratio is similar to that seen after Sr²⁺-induced oocyte activation (Fig.5b). However, the total cell number in the blastocysts does show some dependency upon hPLCζ-luc level. With lower levels of expression (0.1 to 0.4 cps), the total cell number is significant lower than that in blastocysts activated by SrCl₂ (39.8±3.6 versus 63.6±2.5 for 0.1 cps and 40.4±7.4 versus 68.4±2.6 for 0.4 cps; Fig.5a). In contrast, blastocysts induced with higher level of hPLCζ-luc expression (cps 0.6-2.7) have the same cell number as that activated with SrCl₂ (Fig.5a). These data suggest that a specific level of PLCζ-luc expression may be required to achieve an optimal number of cells in a blastocyst.

### The effects of luciferase cRNA injection.

From the experiments described above, it is clear that injection of hPLCζ-luc cRNA can readily activate oocytes but hPLCζ-luc expression can also have a detrimental effect on further development at higher concentrations. In order to establish that these effects are not due to non-specific effects associated with the over-expression of luciferase, or with the injection of exogenous cRNA, we injected mouse oocytes with a control cRNA encoding luciferase protein alone. Mouse oocytes were injected with luciferase cRNA at the same maximal concentration that we used for hPLC4-luciferase (0.5µg/µl). These luciferase-injected oocytes were then subsequently activated either by incubation with SrCl₂ or by hPLCζ-luc injection (0.1 µg/µl) and cultured in KSOM medium. As shown in Figure 6a, the average luminescence from these luciferase-injected oocytes was 19 ± 5.2 cps, which is much greater than the maximal signals we obtained with hPLCζ-luc injection alone (Fig.1). Despite the high level of luciferase expression, we found that the blastocyst formation rate of these oocytes was not different from the control oocytes activated by either SrCl₂, or by a single injection of 0.1 µg/µl hPLCζ-luc (Fig.6b). These results indicate that neither luciferase, nor exogenous RNA injection, can account for any attenuation of development potential of hPLCζ-luc-activated oocytes.

### The effects of hPLCζ-luc in human oocytes.

As reported previously for hPLC, injection hPLCζ-luc cRNA leads to the functional expression of hPLCζ-luc protein in human oocytes (Fig.7). The expression profile of hPLCζ-luc protein was monitored over ∼15 hours and it can be seen that there was a steady increase in luminescence suggesting that hPLCζ-luc is progressively synthesized in human oocytes in a similar way to that shown in mouse oocytes. These data suggest that human oocytes behave in a similar manner to model mouse oocytes with regards to the expression of hPLCζ-luc. However, the different traces in Fig 7a and 7b illustrate that the pattern of expression was somewhat more variable than that we have seen in mouse oocytes. Fig.8 illustrates that injection of hPLCζ-luc cRNA also causes the induction of Ca²⁺ oscillations in human oocytes. The pattern of Ca²⁺ oscillations is shown for 5 different human oocytes displaying different levels of hPLCζ-luc expression. The expression level is indicated in terms of the luminescence level (in counts per second) from each oocyte. As was observed with mouse oocytes, there is a tendency for human oocytes to show higher frequency Ca²⁺ oscillations with higher levels of hPLCζ-luc, as well as a tendency for Ca²⁺ oscillations to stop prematurely when hPLCζ-luc levels were very high (Fig.8). Again, we noted that there is more variability with human oocytes in the pattern of Ca²⁺ oscillations when compared to mouse oocytes (Fig. 8). Nevertheless, the overall response of human oocytes is consistent with parallel studies in mouse oocytes.

The activation and developmental progression rate of human embryos injected with hPLCζ-luc RNA was monitored in a total of 78 human oocytes (26 fresh, 52 aged oocytes) that were obtained from the IVF clinic over a period of 9 months. The expression level in all embryos was monitored at 15-189 hours after injection of hPLCζ-luc by measuring the luminescence. Only a small proportion of embryos develop past the first few cleavage divisions and progressed to the morula or blastocyst stages (Fig.8). However, a small number of human embryos activated by hPLCζ-luc did develop up to the blastocyst stage (Fig.8). It was noticeable that the expression level of hPLCζ-luc determined in these embryos, following activation, was within a narrow range of 1.6-3.9 cps. We have calibrated the levels of luciferase protein in oocytes by injecting known amounts of recombinant luciferase protein. With the microscope and camera system we used, and with 1 mM luciferin, we have determined that 1 cps of luminescence corresponds to about 106 fg of injected luciferase protein in an oocyte. This implies that the amount of human PLCζ protein that is consistent with development to the blastocyst stage is in the range of 170-410 fg (1.6-3.9 cps). Our data, therefore, suggests that this restricted range of hPLCζ expression is a necessary condition for effective development of human embryos to the blastocyst stage. This conclusion is consistent with the extensive studies on mouse oocytes, where we also noted that there was about a 4-fold range of hPLCζ-luc expression level that was permissive for development to the blastocyst stage.

### Discussion

In this study, we have injected cRNA encoding the human PLCζ fused with luciferase into mouse and human eggs to investigate the most effective range over which the PLCζ can stimulate development up to the blastocyst stage. We previously reported that human PLCζ cRNA injection stimulates mouse and human oocyte activation and can lead to embryo development to the blastocyst stage (Cox *et al.,* 2002; Rogers *et al.,* 2004). However, these studies did not measure the amount of protein expression associated with the cRNA injection method and so the precise relationship between PLCζ, Ca²⁺ oscillations and embryo development had not been quantitatively assessed. We have enabled direct measurement of PLCζ protein in the current study by monitoring the luminescence of luciferase that has been fused to the C-terminus of the HPLCζ. The suitability of this method for studying development is vindicated since control experiments showed that neither the oocyte microinjection procedure nor expression of luciferase protein impairs Sr²⁺- or PLCζ-induced embryo development to the blastocsyst stage in mouse (Fig.6). Consequently, we can conclude that the observed effects on Ca²⁺ oscillations and embryo development are due to different levels of PLCζ protein expressed in the oocyte, rather than our method of monitoring expression. With this direct quantification, we are able to estimate that about 1 fg of human PLCζ protein can effectively initiate the Ca²⁺ activation signal in mammalian eggs. This 1 fg threshold is slightly less than the estimated 20-50 fg of mouse PLCζ required to trigger Ca²⁺ oscillations (Saunders *et al.*,, 2002) and is consistent with the suggestion that human PLCζ may be more potent than mouse PLCζ (Cox *et al.,* 2002).

Consistent with several previous studies using untagged, or mouse PLCζ, we found that hPLCζ-luc cRNA injection reliably triggered Ca²⁺ oscillations in mouse oocytes. By quantifying hPLCζ level in oocytes, we showed that the higher the level of hPLCζ protein expression the earlier the first Ca²⁺ increase occurs after injection (Fig.2). Increased expression of PLCζ protein also correlates with a higher frequency of Ca²⁺ transients, but a reduction in the duration for the overall Ca²⁺ oscillation response (Figs.2,3). The finding that more rapid PLCζ synthesis correlates with a quicker onset and higher frequency of Ca²⁺ changes is entirely consistent with models of Ca²⁺ oscillations. The enzymatic product of PLCζ is InsP₃, and the level of InsP₃ in a cell determines both the latency and frequency of oscillations (Dupont & Goldbeter 1993; Keizer *et al.,* 1995). A surprising result is the finding that Ca²⁺ oscillations terminate more quickly in oocytes expressing high levels of PLCζ-luc protein (Fig.2). The termination of Ca²⁺ oscillations in these cases occurs during the phase of increasing synthesis of PLCζ, suggesting there is a feedback mechanism regulating the activity of PLCζ, or a desensitization of the oocyte to its effects. Previous work has shown that mouse PLCζ undergoes nuclear localization upon pronuclear formation, and that this nuclear localization may be responsible for the termination of oscillations (Larman *et al.,* 2004). However, this is unlikely to explain the cessation of Ca²⁺ oscillations in the current study since Ca²⁺ increase often stopped well before (1-2 hr) the observation of pronuclear formation (6 hr). Nuclear sequestration of PLCζ may also be limited in capacity and it is unlikely that all the hPLCζ is localized to the nuclei at high expression levels. There may be other mechanisms for termination of Ca²⁺ oscillations involving other aspects of desensitization such as InsP₃ receptor down-regulation or Ca²⁺ store depletion. Regardless of the specific mechanism(s), our data suggests that mouse oocytes possess an innate ability to limit the duration and intensity of Ca²⁺ signals initiated by PLCζ.

The Ca²⁺ oscillations triggered by hPLCζ-luc lead to high rates of oocyte activation as judged by pronuclear formation. This is consistent with previous data using untagged or Venus-tagged PLCζ, and confirms that PLCζ is a very effective activation stimulus for mouse oocytes. The present data demonstrates that activation of oocytes occurs over a wide range of PLCζ levels and that the activation process is not very sensitive to the pattern of Ca²⁺ oscillations apparently requiring only that a sufficient amount of Ca²⁺ release is achieved during the activation phase (Toth *et al.,* 2006). In direct contrast to oocyte activation, we found that subsequent development of mouse embryos is markedly affected by the level of PLCζ-luc expression. Firstly we found that low levels of hPLCζ-luc expression (0.05cps) induced a reasonable number of Ca²⁺ spikes resulting in high rates of oocyte activation (95%, Fig. 4), however this did not lead to any development to the blastocyst stage. Moreover, oocytes expressing high levels of PLCζ-luc also resulted in Ca²⁺ oscillations and oocyte activation, but these similarly failed to develop further to the blastocyst stage. These data strongly implies that, in contrast to oocyte activation, there is a relatively narrow window of PLCζ expression required to trigger successful development to the blastocyst stage in mammals.

Our results have implications for our understanding of male factor infertility. If the fertilization process proceeds with an otherwise normal human sperm but which contains little or no PLCζ, we would expect that successful oocyte activation would not occur. It is possible that such extreme cases of PLCζ deficiency could account for previously documented cases of failed oocyte activation after ICSI. It is in these potential cases of PLCζ deficiency where fertilization may be rescued by the application of stimuli, including PLCζ, that can generate a Ca²⁺ increase. However, our current study raises the possibility of more subtle developmental effects relevant to putative therapeutic applications of PLCζ for oocyte activation. The effective level of PLCζ expression consistent with good preimplantation development occurs within a narrow window spanning only about a 4 fold concentration range. If the level of functional PLCζ in sperm is abnormally low or high, and hence outside of the required physiological window, it is possible that the fertilizing sperm may cause oocyte activation, but later embryo development does not proceed. These cases would not be noticed as overt activation failure, in ICSI or IVF treatment, and in most clinical scenarios they may only be evident as failures to establish pregnancy. Successful delivery of appropriately calibrated levels of functional PLCζ that triggers both oocyte activation and development to blastocyst may be an effective therapy in these situations.

### Example 2 - Preliminary Study of human PLC-ζ Levels in Fertile and Infertile Sperm

_Normal and infertile human sperm were analysed by SDS-polyacrylamide gel electrophoresis followed by electrophoretic transfer onto a nitrocellulose membrane (5 x 10[6] sperm per lane). The separated human sperm proteins were probed with an antibody raised to the mammalian sperm PLC-zeta protein and immunoreactive sperm proteins were identified following development of the membrane by enhanced chemiluminescence method.

In Figure 10, the upper panel is an immunoblot showing the ∼70kD protein band corresponding to the mobility of human PLC-zeta, indicated by an arrow. The lower panel is a densitometric analysis of the immunoreactive band indicating the relative content of PLC-zeta in infertile human sperm (15%) relative to normal human sperm (100%).

Although these results are of a preliminary nature, this experiment gives a clear indication that the level of human PLCζ is greatly reduced in the infertile sperm compared with the fertile sperm. This corroborates the findings of Example 1.

### References

Cox LJ, Larman MG, Saunders CM, Hashimoto K, Swann K and Lai FA. (2002) Sperm phospholipase Czeta from humans and cynomolgus monkeys triggers Ca2+ oscillations, activation and development of mouse oocytes. Reproduction 124, 611-623.
Dupont G and Goldbeter A (1993) One pool model for Ca2+ oscillations involving Ca2+ and inositol 1,4,5-trisphosphate as co-agonists for Ca2+ release. Cell Calcium 14, 311-322.
Keizer J, Li YX, Stojilkovic S and Rinzel J (1995) InsP3-induced Ca2+ excitability of the endoplasmic reticulum. Mol Biol Cell. 6, 945-951.
Knott JG, Kurokawa M, Fissore RA, Schultz RM and Williams CJ (2005) Transgenic RNA interference reveals role for mouse sperm phospholipase Czeta in triggering Ca2+ oscillations during fertilization. Biol Reprod 72, 992-996.
Kouchi Z, Fukami K, Shikano T, Oda S, Nakamura Y, Takenawa T and Miyazaki S (2004) Recombinant phospholipase C zeta has high Ca2+ sensitivity and induces Ca2+ oscillations in mouse eggs. J Biol Chem 279, 10408-10412.
Larman MG, Saunders CM, Carroll J, Lai FA and Swann K (2004) Cell cycle-dependent Ca2+ oscillations in mouse embryos are regulated by nuclear targeting of PLCzeta. J Cell Sci 117, 2513-2521.
Nomikos M, Blayney LM, Larman MG, Campbell K, Rossbach A, Saunders CM, Swann K, Lai FA (2005) Role of phospholipase C-zeta domains in Ca2+-dependent phosphatidylinositol 4,5-bisphosphate hydrolysis and cytoplasmic Ca2+ oscillations. J Biol Chem 280, 31011-31018.
Rogers NT, Hobson E, Pickering S, Lai FA, Braude P and Swann K (2004) Phospholipase Czeta causes Ca2+ oscillations and parthenogenetic activation of human oocytes. Reproduction 128, 697-702.
Saunders CM, Larman MG, Parrington J, Cox LJ, Royse J, Blayney LM, Swann K and Lai FA (2002) PLC zeta: a sperm-specific trigger of Ca2+ oscillations in eggs and embryo development. Development 129, 3533-3544
Toth S, Huneau D, Banrezes B and Ozil JP. (2006) Egg activation is the result of calcium signal summation in the mouse. Reproduction 131, 27-34.
Van Soom A, Vanroose G, and de Kruif A (2001) Blastocyst evaluation by means of differential staining: a practical approach. Reprod Domest Anim 36, 29-35.
Campbell, K. & Swann,K. (2006) Ca2+ oscillations stimulate an ATP increase during fertilization of mouse eggs. Devel. Biol. 298, 225-233.
Yu, Y., Saunders, C.M., Lai, F.A. and Swann, K. (2008) PLCζ -dependence of preimplantation development in activated mouse oocytes, Hum Reprod 23(2):365-73.

## Claims

1. A composition comprising human PLCζ and a pharmaceutically acceptable excipient or carrier, wherein the composition is a fertilisation fluid for injecting into a human oocyte and said fluid is **characterised in that** the injected volume contains between 170 and 410 femtograms of human PLCζ.

2. An *in vitro* fertilisation method comprising fertilising a human oocyte with sperm obtained from a human male wherein the concentration of human PLCζ per sperm cell has been determined to be less than 170 femtograms; and injecting the oocyte with human PLCζ such that the amount of human PLCζ in the oocyte after injection is between 170 and 410 femtograms.

3. A method as claimed in claim 2 wherein the oocyte is injected with human PLCζ before, after or simultaneously with the sperm.

4. A method as claimed in claim 2 or claim 3 wherein the fertilisation step is carried out by ICSI.

5. A diagnostic or screening method for determining the fertility status of a human male comprising:
a) using a sample of sperm obtained from said human male to determine the concentration of human PLCζ contained therein by:
b) measuring the amount of human PLCζ in each sperm cell and where the concentration is less than 170 femtograms;
c) concluding that the male is likely to be infertile.

6. A diagnostic or screening method for determining the fertility status of a human male comprising:
a) using a sample of sperm obtained from said human male to determine the concentration of human PLCζ contained therein by:
b) measuring the amount of human PLCζ in each sperm cell and where the concentration is more than 410 femtograms;
c) concluding that the male is likely to be infertile.

## Patentansprüche

1. Zusammensetzung, umfassend menschliches PLCζ und einen pharmazeutisch annehmbaren Hilfsstoff oder Träger, wobei die Zusammensetzung eine Fertilisationsfluid zum Injizieren in eine menschliche Oozyte ist und wobei das Fluid **dadurch gekennzeichnet ist, dass** da injizierte Volumen zwischen 170 und 410 Femtogramm humanes PLCζ enthält.

2. In-vitro-Fertilisationsverfahren, umfassend Fertilisieren einer menschlichen Oozyte mit Sperma, das von einem männlichen Menschen erhalten wurde, wobei die Konzentration von humanem PLCζ pro Spermzelle zu weniger als 170 Femtogramm bestimmt wurde; und Injizieren humanes PLCζ in die Oozyte, so dass die Menge an humanem PLCζ in der Oozyte nach Injektion zwischen 170 und 410 Femtogramm ist.

3. Verfahren nach Anspruch 2, wobei die Oozyte nach oder gleichzeitig mit dem Sperma mit humanem PLCζ injiziert wird.

4. Verfahren nach Anspruch 2 oder Anspruch 3, wobei der Fertilisationsschritt durch ICSI durchgeführt wird.

5. Diagnostik- oder Screening-Verfahren zur Bestimmung des Fertilisationsstatus eines männlichen Menschen, umfassend:
a) Verwenden einer von dem männlichen Menschen erhaltenen Spermaprobe zur Bestimmung der Konzentration von darin enthaltenem humanem PLCζ durch;
b) Messen der Menge an humanem PLCζ in jeder Spermzelle und wobei die Konzentration weniger als 170 Femtogramm beträgt;
c) Feststellen, dass der Mann wahrscheinlich unfruchtbar ist.

6. Diagnostik- oder Screening-Verfahren zur Bestimmung des Fertilisationsstatus eines männlichen Menschen, umfassend:
a) Verwenden einer von dem männlichen Menschen erhaltenen Spermaprobe zur Bestimmung der Konzentration von darin enthaltenem humanem PLCζ durch;
b) Messen der Menge an humanem PLCζ in jeder Spermzelle und wobei die Konzentration mehr als 410 Femtogramm beträgt;
c) Feststellen, dass der Mann wahrscheinlich unfruchtbar ist.

## Revendications

1. Composition comprenant la PLCζ humaine et un excipient ou support pharmaceutiquement acceptable, la composition étant un liquide de fertilisation devant être injecté dans un ovocyte humain et ledit liquide étant **caractérisé en ce que** le volume injecté contient entre 170 et 410 femtogrammes de PLCζ humaine.

2. Procédé de fertilisation in vitro comprenant la fertilisation d'un ovocyte humain avec du sperme provenant d'un homme, la concentration de la PLCζ humaine par spermatozoïde a été déterminée comme étant inférieure à 170 et l'injection de l'ovocyte avec de la PLCζ humaine de telle sorte que la quantité de la PLCζ humaine dans l'ovocyte après l'injection soit entre 170 et 410 femtogrammes.

3. Procédé selon la revendication 2, dans lequel la PLCζ humaine est injectée dans l'ovocyte avant, après ou en même temps que le sperme.

4. Procédé selon la revendication 2 ou la revendication 3, dans lequel l'étape de fertilisation est réalisée par ISIC.

5. Procédé de diagnostic ou de dépistage permettant de déterminer l'état de fertilité d'un homme comprenant :
a) l'utilisation d'un échantillon de sperme provenant dudit homme afin de déterminer la concentration en PLCζ humaine contenue dans l'échantillon ;
b) la mesure de la quantité de PLCζ humaine dans chaque spermatozoïde et ou la concentration est inférieure à 170 femtogrammes ;
c) la conclusion que l'homme est probablement stérile.

6. Procédé de diagnostic ou de dépistage permettant de déterminer l'état de fertilité d'un homme comprenant :
a) l'utilisation d'un échantillon de sperme provenant dudit homme afin de déterminer la concentration en PLCζ humaine contenue dans échantillon ;
b) la mesure de la quantité de PLCζ humaine dans chaque spermatozoïde et la concentration est supérieure à 410 femtogrammes ;
c) la conclusion que l'homme est probablement stérile.


### SEQUENCE LISTING

<110> University College Cardiff Consultants Limited
<120> Fertilisation Protein
<130> UC3.31 WO
<150> GB 0715325
   <151> 2007-08-08
<160> 2
<170> PatentIn version 3.1
<210> 1
   <211> 1827
   <212> DNA
   <213> Homo sapiens
<220>
   <221> CDS
   <222> (1)..(1827)
   <223>
<400> 1
   atg gaa atg aga tgg ttt ttg tca aag att cag gat gac ttc aga ggt
   48
   Met Glu Met Arg Trp Phe Leu Ser Lys Ile Gln Asp Asp Phe Arg Gly
   1               5                   10                  15
   gga aaa att aac cta gaa aaa act cag agg tta ctt gaa aaa tta gat
   96
   Gly Lys Ile Asn Leu Glu Lys Thr Gln Arg Leu Leu Glu Lys Leu Asp
               20                  25                  30
   att cgg tgc agt tat att cat gtg aaa cag att ttt aag gac aat gac
   144
   Ile Arg Cys Ser Tyr Ile His Val Lys Gln Ile Phe Lys Asp Asn Asp
           35                  40                  45
   agg ctg aaa caa gga aga atc acc ata gaa gaa ttt aga gca att tat
   192
   Arg Leu Lys Gln Gly Arg Ile Thr Ile Glu Glu Phe Arg Ala Ile Tyr
       50                  55                  60
   cga att atc acg cac aga gaa gaa att att gag att ttc aac aca tat
   240
   Arg Ile Ile Thr His Arg Glu Glu Ile Ile Glu Ile Phe Asn Thr Tyr
   65                  70                  75                  80
   tct gaa aac cgg aaa att ctt tta gca agt aat ctg gct caa ttt ctg
   288
   Ser Glu Asn Arg Lys Ile Leu Leu Ala Ser Asn Leu Ala Gln Phe Leu
                   85                  90                  95
   aca caa gaa caa tat gca gct gag atg agt aaa gct att gct ttt gag
   336
   Thr Gln Glu Gln Tyr Ala Ala Glu Met Ser Lys Ala Ile Ala Phe Glu
               100                 105                 110
   atc att cag aaa tac gag cct atc gaa gaa gtt agg aaa gca cac caa
   384
   Ile Ile Gln Lys Tyr Glu Pro Ile Glu Glu Val Arg Lys Ala His Gln
           115                 120                 125
   atg tca tta gaa ggt ttt aca aga tac atg gat tca cgt gaa tgt cta
   432
   Met Ser Leu Glu Gly Phe Thr Arg Tyr Met Asp Ser Arg Glu Cys Leu
       130                 135                 140
   ctg ttt aaa aat gaa tgt aga aaa gtt tat caa gat atg act cat cca
   480
   Leu Phe Lys Asn Glu Cys Arg Lys Val Tyr Gln Asp Met Thr His Pro
   145                 150                 155                 160
   tta aat gat tat ttt att tca tct tca cat aac aca tat ttg gta tct
   528
   Leu Asn Asp Tyr Phe Ile Ser Ser Ser His Asn Thr Tyr Leu Val Ser
                   165                 170                 175
   gat caa tta ttg gga cca agt gac ctt tgg gga tat gta agt gcc ctt
   576
   Asp Gln Leu Leu Gly Pro Ser Asp Leu Trp Gly Tyr Val Ser Ala Leu
               180                 185                 190
   gtg aaa gga tgc cgt tgt ttg gag att gac tgc tgg gat gga gca caa
   624
   Val Lys Gly Cys Arg Cys Leu Glu Ile Asp Cys Trp Asp Gly Ala Gln
           195                 200                 205
   aat gaa cct gtt gta tat cat ggc tac aca ctc aca agc aaa ctt ctg
   672
   Asn Glu Pro Val Val Tyr His Gly Tyr Thr Leu Thr Ser Lys Leu Leu
       210                 215                 220
   ttt aaa act gtt atc caa gct ata cac aag tat gca ttc atg aca tct
   720
   Phe Lys Thr Val Ile Gln Ala Ile His Lys Tyr Ala Phe Met Thr Ser
   225                 230                 235                 240
   gac tac cca gtg gtg ctc tct tta gaa aat cac tgc tcc act gcc caa
   768
   Asp Tyr Pro Val Val Leu Ser Leu Glu Asn His Cys Ser Thr Ala Gln
                   245                 250                 255
   caa gaa gta atg gca gac aat ttg cag gct act ttt gga gag tcc ttg
   816
   Gln Glu Val Met Ala Asp Asn Leu Gln Ala Thr Phe Gly Glu Ser Leu
               260                 265                 270
   ctt tct gat atg ctt gat gat ttt cct gat act cta cca tca cca gag
   864
   Leu Ser Asp Met Leu Asp Asp Phe Pro Asp Thr Leu Pro Ser Pro Glu
           275                 280                 285
   gca cta aaa ttc aaa ata tta gtt aaa aat aag aaa ata gga acc tta
   912
   Ala Leu Lys Phe Lys Ile Leu Val Lys Asn Lys Lys Ile Gly Thr Leu
       290                 295                 300
   aag gaa acc cat gaa aga aaa ggt tct gat aag cgt gga gac aat caa
   960
   Lys Glu Thr His Glu Arg Lys Gly Ser Asp Lys Arg Gly Asp Asn Gln
   305                 310                 315                 320
   gac aag gaa aca ggg gta aaa aag tta cct gga gta atg ctt ttc aag
   1008
   Asp Lys Glu Thr Gly Val Lys Lys Leu Pro Gly Val Met Leu Phe Lys
                   325                 330                 335
   aaa aag aag acc agg aag cta aaa att gct ctg gcc tta tct gat ctt
   1056
   Lys Lys Lys Thr Arg Lys Leu Lys Ile Ala Leu Ala Leu Ser Asp Leu
               340                 345                 350
   gtc att tat acg aaa gct gag aaa ttc aaa agc ttt caa cat tca aga
   1104
   Val Ile Tyr Thr Lys Ala Glu Lys Phe Lys Ser Phe Gln His Ser Arg
           355                 360                 365
   tta tat cag caa ttt aat gaa aat aat tct att ggg gag aca caa gcc
   1152
   Leu Tyr Gln Gln Phe Asn Glu Asn Asn Ser Ile Gly Glu Thr Gln Ala
       370                 375                 380
   cga aaa ctt tca aaa ttg cga gtc cat gag ttt att ttt cac acc agg
   1200
   Arg Lys Leu Ser Lys Leu Arg Val His Glu Phe Ile Phe His Thr Arg
   385                 390                 395                 400
   aag ttc att acc aga ata tat ccc aaa gca aca aga gca gac tct tct
   1248
   Lys Phe Ile Thr Arg Ile Tyr Pro Lys Ala Thr Arg Ala Asp Ser Ser
                   405                 410                 415
   aat ttt aat ccc caa gaa ttt tgg aat ata ggt tgt caa atg gtg gct
   1296
   Asn Phe Asn Pro Gln Glu Phe Trp Asn Ile Gly Cys Gln Met Val Ala
               420                 425                 430
   tta aat ttc cag acc cct ggt ctg ccc atg gat ctg caa aat ggg aaa
   1344
   Leu Asn Phe Gln Thr Pro Gly Leu Pro Met Asp Leu Gln Asn Gly Lys
           435                 440                 445
   ttt ttg gat aat ggt ggt tct gga tat att ttg aaa cca cat ttc tta
   1392
   Phe Leu Asp Asn Gly Gly Ser Gly Tyr Ile Leu Lys Pro His Phe Leu
       450                 455                 460
   aga gag agt aaa tca tac ttt aac cca agt aac ata aaa gag ggt atg
   1440
   Arg Glu Ser Lys Ser Tyr Phe Asn Pro Ser Asn Ile Lys Glu Gly Met
   465                 470                 475                 480
   cca att aca ctt aca ata agg ctc atc agt ggt atc cag ttg cct ctt
   1488
   Pro Ile Thr Leu Thr Ile Arg Leu Ile Ser Gly Ile Gln Leu Pro Leu
                   485                 490                 495
   act cat tca tca tct aac aaa ggt gat tca tta gta att ata gaa gtt
   1536
   Thr His Ser Ser Ser Asn Lys Gly Asp Ser Leu Val Ile Ile Glu Val
               500                 505                 510
   ttt ggt gtt cca aat gat caa atg aag cag cag act cgt gta att aaa
   1584
   Phe Gly Val Pro Asn Asp Gln Met Lys Gln Gln Thr Arg Val Ile Lys
           515                 520                 525
   aaa aat gct ttt agt cca aga tgg aat gaa aca ttc aca ttt att att
   1632
   Lys Asn Ala Phe Ser Pro Arg Trp Asn Glu Thr Phe Thr Phe Ile Ile
       530                 535                 540
   cat gtc cca gaa ttg gca ttg ata cgt ttt gtt gtt gaa ggt caa ggt
   1680
   His Val Pro Glu Leu Ala Leu Ile Arg Phe Val Val Glu Gly Gln Gly
   545                 550                 555                 560
   tta ata gca gga aat gaa ttt ctt ggg caa tat act ttg cca ctt cta
   1728
   Leu Ile Ala Gly Asn Glu Phe Leu Gly Gln Tyr Thr Leu Pro Leu Leu
                   565                 570                 575
   tgc atg aac aaa ggt tat cgt cgt att cct ctg ttt tcc aga atg ggt
   1776
   Cys Met Asn Lys Gly Tyr Arg Arg Ile Pro Leu Phe Ser Arg Met Gly
               580                 585                 590
   gag agc ctt gag cct gct tca ctg ttt gtt tat gtt tgg tac gtc aga
   1824
   Glu Ser Leu Glu Pro Ala Ser Leu Phe Val Tyr Val Trp Tyr Val Arg
           595                 600                 605
   taa
   1827
<210> 2
   <211> 608
   <212> PRT
   <213> Homo sapiens
<400> 2
   Met Glu Met Arg Trp Phe Leu Ser Lys Ile Gln Asp Asp Phe Arg Gly
   1               5                   10                  15
   Gly Lys Ile Asn Leu Glu Lys Thr Gln Arg Leu Leu Glu Lys Leu Asp
               20                  25                  30
   Ile Arg Cys Ser Tyr Ile His Val Lys Gln Ile Phe Lys Asp Asn Asp
           35                  40                  45
   Arg Leu Lys Gln Gly Arg Ile Thr Ile Glu Glu Phe Arg Ala Ile Tyr
       50                  55                  60
   Arg Ile Ile Thr His Arg Glu Glu Ile Ile Glu Ile Phe Asn Thr Tyr
   65                  70                  75                  80
   Ser Glu Asn Arg Lys Ile Leu Leu Ala Ser Asn Leu Ala Gln Phe Leu
                   85                  90                  95
   Thr Gln Glu Gln Tyr Ala Ala Glu Met Ser Lys Ala Ile Ala Phe Glu
               100                 105                 110
   Ile Ile Gln Lys Tyr Glu Pro Ile Glu Glu Val Arg Lys Ala His Gln
           115                 120                 125
   Met Ser Leu Glu Gly Phe Thr Arg Tyr Met Asp Ser Arg Glu Cys Leu
       130                 135                 140
   Leu Phe Lys Asn Glu Cys Arg Lys Val Tyr Gln Asp Met Thr His Pro
   145                 150                 155                 160
   Leu Asn Asp Tyr Phe Ile Ser Ser Ser His Asn Thr Tyr Leu Val Ser
                   165                 170                 175
   Asp Gln Leu Leu Gly Pro Ser Asp Leu Trp Gly Tyr Val Ser Ala Leu
               180                 185                 190
   Val Lys Gly Cys Arg Cys Leu Glu Ile Asp Cys Trp Asp Gly Ala Gln
           195                 200                 205
   Asn Glu Pro Val Val Tyr His Gly Tyr Thr Leu Thr Ser Lys Leu Leu
       210                 215                 220
   Phe Lys Thr Val Ile Gln Ala Ile His Lys Tyr Ala Phe Met Thr Ser
   225                 230                 235                 240
   Asp Tyr Pro Val Val Leu Ser Leu Glu Asn His Cys Ser Thr Ala Gln
                   245                 250                 255
   Gln Glu Val Met Ala Asp Asn Leu Gln Ala Thr Phe Gly Glu Ser Leu
               260                 265                 270
   Leu Ser Asp Met Leu Asp Asp Phe Pro Asp Thr Leu Pro Ser Pro Glu
           275                 280                 285
   Ala Leu Lys Phe Lys Ile Leu Val Lys Asn Lys Lys Ile Gly Thr Leu
       290                 295                 300
   Lys Glu Thr His Glu Arg Lys Gly Ser Asp Lys Arg Gly Asp Asn Gln
   305                 310                 315                 320
   Asp Lys Glu Thr Gly Val Lys Lys Leu Pro Gly Val Met Leu Phe Lys
                   325                 330                 335
   Lys Lys Lys Thr Arg Lys Leu Lys Ile Ala Leu Ala Leu Ser Asp Leu
               340                 345                 350
   Val Ile Tyr Thr Lys Ala Glu Lys Phe Lys Ser Phe Gln His Ser Arg
           355                 360                 365
   Leu Tyr Gln Gln Phe Asn Glu Asn Asn Ser Ile Gly Glu Thr Gln Ala
       370                 375                 380
   Arg Lys Leu Ser Lys Leu Arg Val His Glu Phe Ile Phe His Thr Arg
   385                 390                 395                 400
   Lys Phe Ile Thr Arg Ile Tyr Pro Lys Ala Thr Arg Ala Asp Ser Ser
                   405                 410                 415
   Asn Phe Asn Pro Gln Glu Phe Trp Asn Ile Gly Cys Gln Met Val Ala
               420                 425                 430
   Leu Asn Phe Gln Thr Pro Gly Leu Pro Met Asp Leu Gln Asn Gly Lys
           435                 440                 445
   Phe Leu Asp Asn Gly Gly Ser Gly Tyr Ile Leu Lys Pro His Phe Leu
       450                 455                 460
   Arg Glu Ser Lys Ser Tyr Phe Asn Pro Ser Asn Ile Lys Glu Gly Met
   465                 470                 475                 480
   Pro Ile Thr Leu Thr Ile Arg Leu Ile Ser Gly Ile Gln Leu Pro Leu
                   485                 490                 495
   Thr His Ser Ser Ser Asn Lys Gly Asp Ser Leu Val Ile Ile Glu Val
               500                 505                 510
   Phe Gly Val Pro Asn Asp Gln Met Lys Gln Gln Thr Arg Val Ile Lys
           515                 520                 525
   Lys Asn Ala Phe Ser Pro Arg Trp Asn Glu Thr Phe Thr Phe Ile Ile
       530                 535                 540
   His Val Pro Glu Leu Ala Leu Ile Arg Phe Val Val Glu Gly Gln Gly
   545                 550                 555                 560
   Leu Ile Ala Gly Asn Glu Phe Leu Gly Gln Tyr Thr Leu Pro Leu Leu
                   565                 570                 575
   Cys Met Asn Lys Gly Tyr Arg Arg Ile Pro Leu Phe Ser Arg Met Gly
               580                 585                 590
   Glu Ser Leu Glu Pro Ala Ser Leu Phe Val Tyr Val Trp Tyr Val Arg
           595                 600                 605